Europäisches Patentamt

European Patent Office

Office européen des brevets

(19)

(11) Publication number: **0 120 631**
**B1**

# (12) EUROPEAN PATENT SPECIFICATION

(45) Date of publication of patent specification: **22.04.87**

(21) Application number: **84301485.3**

(22) Date of filing: **06.03.84**

(51) Int. Cl.⁴: **C 07 C 51/12, C 07 C 53/08,
C 07 C 67/36, C 07 C 69/14**

(54) **A process for producing carboxylic acids by carbonylation of alkanols over a carbon catalyst.**

(30) Priority: **25.03.83 US 478829
25.03.83 US 478828
25.03.83 US 478830**

(43) Date of publication of application:
**03.10.84 Bulletin 84/40**

(45) Publication of the grant of the patent:
**22.04.87 Bulletin 87/17**

(84) Designated Contracting States:
**DE FR GB IT NL**

(56) References cited:
**EP-A-0 033 212
EP-A-0 069 514
WO-A-81/00856
DE-A-1 965 239**

(73) Proprietor: **TEXACO DEVELOPMENT
CORPORATION
2000 Westchester Avenue
White Plains New York 10650 (US)**

(72) Inventor: **Larkin, John Michael
12414 Dorsett Road
Austin Texas 78759 (US)**
Inventor: **Duranleau, Roger George
Route 5 Box 87
Georgetown Texas 78626 (US)**
Inventor: **Estes, John Harold
RFD 6 Cedar Hill Road
Wappingers Falls, N.Y. 12590 (US)**

(74) Representative: **Burnside, Michael et al
Michael Burnside & Partners 2 Serjeants' Inn
Fleet Street
London EC4Y 1HL (GB)**

Courier Press, Leamington Spa, England.

**0 120 631**

**Description**

Acetic acid has been known as an industrial chemical for many years, and large amounts are used in the manufacture of various other chemical products. Proposals for producing carboxylic acids by the action of carbon monoxide upon alcohols (carbonylation) have been disclosed in the art.

Carbonylation of methanol has been performed industrially in the liquid phase under extremely high pressure (ca. 40 MPa) using an iodide or carbonyl of cobalt as catalyst, and an organic or inorganic iodide, such as methyl iodide, hydrogen iodide, or potassium iodide, as promoter. Recently, it has been reported that nickel carbonyl or other nickel compounds are effective catalysts for carbonylation of methanol in the presence of iodide at pressure as low as 3 MPa, when some organic amines or phosphines are incorporated in the liquid reaction media; (DE—A—2749955 and JP—54-59211).

Roth and Paulik (Roth et al. *Chemtech* 1971, 600; and Paulik and Roth *Chem. Commun.* 1968, 1578) have found that carbonyl complexes of rhodium are exceedingly active for methanol carbonylation in the presence of iodide promoters. They are sufficiently active at atmospheric pressure and selectively give methyl acetate and acetic acid. Extensive work on catalysis by rhodium has clarified the mechanism, which comprises oxidative addition of methyl iodide to rhodium, insertion of CO, and reductive decomposition of the resulting acyl complex by methanol or water, (Forster *J. Am. Chem. Soc.* 1976 *98*, 846; or Hjortkjaer and Jensen *Ind. Eng. Chem. Prod. Res. Dev.* 1976 *15*, 46). Rhodium is also an effective catalyst for the vapour phase carbonylation of methanol when it is supported on activated carbon or metal oxides by an impregnation method, (Schultz and Montgomery *J. Catal.* 1969 *13*, 105; Robinson et al. *J. Catal.* 1972 *27*, 380; or Krzywicki and Marczewsi *J. Mol. Catal.* 1979 *6*, 431); or on zeolite by means of an ion exchange method (Yashima et al. *J. Catal.* 1979 *59*, 53; Andersson and Scurrel *J. Catal.* 1979 *59*, 340; or Scurrell and Howe *J. Mol. Catal.* 1980 *7*, 535).

In FR—A—2 030 118, a process is disclosed wherein carboxylic acids, especially acetic acid, are produced by reacting methanol and carbon monoxide over an activated carbon bed at a temperature of 200 to 500°C and a pressure of from 0.75 to 21 MPa, using a halogen promoter which is either dispersed on the carbon bed or incorporated as a component of the catalyst system. In this process, the activation of the carbon, which can optionally be washed with an HF or HNO₃ solution, is accomplished by heating the carbon in a stream of nitrogen at 0.1 MPa for 1 to 2 hours.

Disadvantages of this system include the use of generally higher temperatures, deactivation of the system within a relatively short time, the need to activate the carbon bed with nitrogen for best results, and no apparent reported difference on results using various different types of carbon bed.

In the *Indian Journal of Technology,* Vol. 5, 266 (1967), a process is disclosed for synthesizing methyl formate from methanol and carbon monoxide in the presence of alkali-activated charcoal catalysts. Although an increase in pressure was reported to increase conversion, temperatures above 150°C caused a decrease in conversion.

WO 81/00856 (EP—A—0039354) discloses the use of halogen-promoted supported ruthenium catalysts for the carbonylation of alcohols to carboxylic acids, the catalyst support being one of a variety of organic and inorganic supports, including active carbon.

EP—A—0069514 discloses the carboxylation of alcohols in the presence of nickel or cobalt catalysts supported on a carbonaceous substrate, e.g. activated carbon, carbon black or coke.

It is an object of the present invention to provide a system for methanol carbonylation where there is higher conversion from methanol, higher selectivity for acetic acid, where the catalyst has a longer life and where carboxylic acids are produced continuously. An additional object of this invention is to provide a system which affords economical advantages in that the system can operate at a lower temperature without special addition of metal catalysts. Finally, an object of this invention is to optimize results by use of particular types of activated carbon bed having a particular pore size.

The present invention provides a process for the continuous production of carboxylic acids and esters by the reaction of carbon monoxide with an alkanol, at a temperature of from 240 to 300°C and a pressure of 3.5 to 27.5 MPa, in the presence of a halide promoter activated carbon, and in the presence of at least 1 ppm, preferably from 25 to 150 ppm, of a transition metal selected from cobalt, ruthenium, iron, nickel, rhodium, palladium, osmium, iridium and platinum.

The activated carbon which is used according to the invention is derived from coal or peat and has a density from 0.03 to 2.5 cm³/g, a surface area from 200 to 2000 m²/g, and a pore size from 0.6 to 200 nm.

According to one preferred embodiment, the reaction takes place at a pressure from 5 to 27.5 MPa in the presence of a halide, carboxylate, nitrate or carbonyl of the transition metal.

In an alternative embodiment, the reaction takes place at a pressure of from 3.5 to 21 MPa, the transition metal resulting from the reaction being conducted in a nickel or cobalt alloy reactor.

The halide promoter is preferably an alkyl halide, hydrogen halide, acyl halide or dihalomethane.

If desired, the reaction is carried out in the presence of a solvent.

In a further embodiment, the catalyst can be reactivated by contact with steam at a temperature of from 500 to 1500°C and a pressure of from 0.1 to 0.5 MPa for a period of from 1 to 25 hours, preferably in the presence of an activating agent selected from oxides, hydroxides and halides of alkali metals or alkaline earth metals, inorganic sulphur compounds and mineral acids.

Catalysts that are suitable for the practice of this invention contain a soluble transition metal, either added as such or resulting from the material from which the reactor is constructed. Also necessary for this invention, is a bed of activated carbon which can optionally be washed. The soluble metal catalyst may be chosen from a wide variety of organic or inorganic compounds, complexes, etc., as will be shown and illustrated below. It is only necessary that the catalyst precursor actually employed contain certain Group VIII metals in a soluble state. It has been found that a halide promoter is necessary for the carbonylation to take place according to the general scheme outlined above.

The actual catalytically-active species is unknown, but is believed to comprise a Group VIII metal in complex combination with a halide promoter, and in association with the activated carbon in the carbon bed.

The soluble metal catalyst precursors may take many different forms. Generally the metal species used are Group VIII transition metals (Co, Ru, Fe, Ni, Rh, Pd, Os, Ir and Pt). The soluble Group VIII metal catalyst may be added to the reaction mixture in the form of a carbonyl, for example, triruthenium dodecacarbonyl, dicobalt octacarbonyl, iron pentacarbonyl, nickel tetracarbonyl, diiron nonacarbonyl or tetracobalt dodecacarbonyl. Alternatively the Group VIII metal may be added as the salt of a mineral acid, for example, ruthenium trichloride, iron(II) iodide, iron(III) nitrate, cobalt(II) nitrate, cobalt(II) chloride or nickel(II) iodide; or as the salt of a suitable organic carboxylic acid, for example, cobalt(II) acetate, cobalt(III) acetate, Ni(II) propionate or Fe(II) naphthenate. The metal may furthermore be added to the reaction zone as a complex with a trisubstituted phosphorus compound, or as a salt of an enolate. Here suitable examples include cobalt(III) 2,4-pentanedionate and dichlorotris(triphenylphosphine)-ruthenium(II).

Preferred Group VIII soluble transition metal catalysts include carbonyls and halides. Among the particularly preferred are ruthenium chloride, ruthenium dodecacarbonyl and dicobalt octacarbonyl. The usefulness of these Group VIII transition metal precursors for acetic acid synthesis is particularly illustrated by the accompanying Examples 1 to 11.

The quantity of soluble transition metal catalyst employed in this embodiment of the present invention is not critical, and may vary over a wide range. Metal concentrations could range from less than 5 (e.g. 1) to greater than 1000 ppm, depending on the activity of the metal species. In general, this improved process is desirably conducted in the presence of a catalytically effective quantity of the active metal species, in conjunction with a halide promoter, and optionally in the presence of a solvent which gives the desired products in reasonable yields. The reaction proceeds when employing as little as about 0.0001 weight percent, and even lesser amounts of Group VIII metal catalyst together with 0.1 to 50 weight percent of a halide promoter, basis the total weight of the reaction mixture. The upper concentration is dictated by a variety of factors including catalyst cost, partial pressures of carbon monoxide, operating temperature etc. A soluble Group VIII metal catalyst concentration of from 0.002 to 0.02 weight % of metal, in conjunction with an alkyl halide promoter concentration of from 5 to 15 mol %, based on the total mols of reaction mixture, is generally desirable in the practice of this embodiment of the invention.

In an alternative embodiment, either the reactor or the conduit system through which liquid is conducted to the reactor, is constructed of cobalt or nickel alloys or mixtures of cobalt, nickel and other metals. In this embodiment, if the reactor is constructed of inert materials, such as tantalum or glass, then the conduit system should be constructed of cobalt alloys, nickel alloys, or mixtures of cobalt and nickel alloys.

Although no metal catalysts are physically added to the catalyst system in this embodiment of this invention, trace amounts of metal catalyst, which enter the reaction system from the walls of the reactor or through feed lines, do enhance catalytic activity, as will be demonstrated in Examples 9 and 10. Even though they are not separately added to the reactor, it has been found that these metals are present in trace amounts in the reactor, and the conversion of methanol into acetic acid does not proceed with as high a degree of effectiveness when a reactor with an inert lining, such as tantalum, is used. The metal catalysts also require a bed of activated carbon, with pores affording high surface area, which is optionally washed in order that carbonylation activity be manifested. The soluble metal catalysts which are present in amounts of at least 1 ppm, e.g. 100 to 200 ppm, or even more, can be inorganic compounds and complexes of inorganic compounds and are preferably compounds containing Group VIII metals in an ionic state. It has been found that a halide promoter is necessary for the carbonylation to take place according to the general scheme outlined above. The halide promoter, in addition to its normal function of promoting the carbonylation reaction, is thought to also aid in dissolving trace amounts of active carbonylation metals from the metal surfaces it contacts.

The actual catalytically active species in this embodiment is believed to comprise trace amounts of Co or Ni metal in complex combination with a halide promoter and in association with the activated peat or coal-derived carbon of high surface area in the carbon bed.

Composition of the alloy may be 0 to 75% of nickel and 0 to 75% of cobalt, with the balance being iron and other metals. The total of cobalt and nickel should exceed 4% for good conversion of alkanol into carboxylic acid. Common metal alloys which allow the practice of this invention are Type 316 stainless steel, Hastealloy C, and UMCo-50. They are described as follows: Type 316 stainless steel contains 10—14% Ni, 16—18% Cr, 2—3% Mo, 2% Mn, 1% Si, less than 1% C, P, and S, and the balance Fe. Hastealloy C is composed of 56% Ni, 17% Mo, 16.5% Cr, 4.5% W, and 6% Fe; UMCo-50 contains 49% Co, 28% Cr, and 21% Fe. Good results are obtained when the amount of Group VIII transition metals present is from 5 to 200 ppm, or even more. The preferred range has been found to be 25 to 150 ppm.

The metals in trace amounts enter the reactor in solution with other reactants, including methanol, and a gas comprising essentially carbon monoxide along with the halide promoter over an activated carbon bed.

The halide found necessary to effect the desired carbonylation should generally be present in an amount of 1 to 20 mol %. A more preferred range of halide promoter concentration is from 5 to 15 mol %. This promoting component of the catalyst system may be a halogen compound that may be introduced into the reaction zone in liquid form, in gaseous form, or dissolved in a suitable solvent or reactant. Satisfactory halide promoters include hydrogen halides, such as hydrogen iodide and gaseous hydriodic acid, alkyl and aryl halides containing 1 to 12 carbon atoms such as methyl iodide, ethyl iodide, 1-iodopropane, 2-iodobutane, 1-iodobutane, methyl bromide, ethyl bromide, iodobenzene and benzyl iodide as well as acyl iodides such as acetyl iodide. Also suitable as halogen components are quaternary ammonium and phosphonium halides; e.g. tetramethylammonium iodide and tetrabutylphosphonium iodide. Alkali metal and alkaline earth metal halides, such as cesium iodide, may also be used.

Alkyl iodide or bromide promoters having 1 to 6 carbon atoms are the preferred coreactants for the carbonylation reaction of this invention. Most preferred are iodomethane, hydrogen iodide and methyl bromide.

The gas which is reacted with the liquid feed and passed over the activated carbon bed in the process of this invention comprises essentially carbon monoxide.

The carbon monoxide may be used in conjunction with up to 90% by volume of one or more other gases. These other gases may include one or more inert gases such as nitrogen, argon and neon. Hydrogen may also be present, but is preferably absent since it is reactive with carbon monoxide, thus subtracting from the efficiency of the carbonylation process.

The activated carbon bed over which the liquid feed stream is passed can be fixed or fluidized, and is prepared from a porous solid. The density of the solid is from 0.03 to 2.5 $cm^3/gm$, more preferably from 0.05 to 1.5 $cm^3/gm$, and the surface area is from 200 to 2000 $m^2/g$. Carbons can be preformed of compacted granules, powders, or particles. Powdered sources are not preferred.

In the process of this invention, it was discovered that, contrary to discussions in prior art, the source of carbon and pore size of carbon in the carbon bed made a distinct difference in selectivity for acetic acid and, along with the use of a generally lower temperature than has been used in the prior art, resulted in a great improvement in catalyst lifetime over procedures of the prior art.

In particular, contrary to statements in the prior art that the source of activated carbon makes no difference in performance of catalyst systems, as long as natural oil sources are not used, it has been found in this invention (and demonstrated by Examples 12, 13 and 16) that peat-derived and coal-derived sources of carbon afford particular advantages with regard to the effectiveness of the system.

In addition to improving the effectiveness by using particular sources of activated carbon, it has been found that the results can be further enhanced by using activated carbons from peat or coal-derived sources, where the material possesses a particular pore size, which pore size has a relationship to observed effectiveness in converting methanol plus CO into acetic acid in the process of this invention. The best results are obtained where the pore size of the carbon source allows for a higher surface area. Wide pores in the range of 0.6 to 200 nm are used. Pores in the range of 2 to 120 nm are preferable.

The activated carbon bed can optionally be washed. If washed, the treatment consists of an HF solution or $HNO_3$ solution, in an amount of 600 to 2000 ml of $HNO_3$ per 500 g carbon, the $HNO_3$ concentration in water being 2 to 30%. If HF is used, the concentration in water should be from 10 to 55%. Washing time may be from 5 minutes to 24 hours. Further, the acid-washed carbon can be washed with $H_2O$ to remove excess acid.

Suitable sources of activated carbon which may be used in the process of this invention are NORIT RB-1 or SORBORNORIT B-3 activated carbon. (NORIT and SORBORNORIT are registered trademarks of the American Norit Company). Another suitable activated carbon which was used was CARBORUNDUM GAC-616GA (CARBORUNDUM is a registered trademark of Kennecott Corporation). These activated carbons, prepared by the manufacturers according to procedures developed by them, were in the form of granules or pellets, and were described as generally having a surface area of 1000 to 1200 $m^2/g$. Methods of manufacturing activated carbon are listed in the book: *Activated Carbon, Manufacture and Regeneration* by A. Yehaskel, Noyes Data Corporation, Park Ridge, New Jersey, 1978.

In US—A—4 032 476 there is described a method for producing granular activated carbon from sub-bituminous coal. The coal is crushed and screened to afford 8—30 mesh granules, which are subsequently mixed with phosphoric or other mineral acid, ground to a powder, formed into pellets by application of pressure, and then granulated to obtain 6—20 mesh grounds. The granules are heated to 450°C, and then converted into activated carbon by heating in an atmosphere of nitrogen and steam at 800 to 900°C for 4 to 5 hours. The surface area of the activated granules is 900 to 1050 $m^2/g$, and the apparent density is about 0.50 $cm^3/g$. Granules so produced are suitable for the practice of this invention. To obtain activated carbon granules which are even more suited for the practice of this invention, the steam activation treatment can be extended to a period beyond 5 hours. The extended steam treatment has the desirable effect of producing carbon granules of lower density, greater pore size, and greater surface area than described above by the method of US—A—4 032 476.

In M. Smisek and S. Cervy, *Active Carbon, Manufacture, Properties and Application,* Elsevier Publishing Co., New York, N.Y., 1974, there is described a general procedure for producing a pelleted form of activated carbon, for example from peat, which is also suitable for the practice of this invention. According to this procedure, powdered peat is carbonized by heating at 300 to 500°C. The carbon powder is mixed with a binding agent (e.g. coal tar or pitch) and extruded through an orifice to produce solid extrudates. The latter are heated at high temperatures (600 to 1000°C) with controlled amounts of steam, air, or carbon dioxide to yield a high surface area extruded carbon suited for use in the fixed bed application of this invention.

Where carbonylation is performed using methanol, acetic acid is prepared in good yield with conversion of methanol ranging from 10 to 100% and selectivity to acetic acid reaching as high as 100%.

A solvent is generally not necessary for the practice of this invention, since the low molecular weight alkanols which are reactants serve as satisfactory solvents. A solvent could be used, however, if the transition metal compound or the halide promoter were only partially soluble in the alkanol. In other instances, a solvent might be used to aid in separation of products, for example by codistillation or by formation of an insoluble product layer. In still other instances, a solvent might be used to aid in dissipation of heat during the exothermic reaction of carbon monoxide with alkanols to form carboxylic acids. Solvents which could be used include compounds which are inert to the reaction conditions and which do not impede the desired reaction. Suitable solvents include aliphatic or aromatic hydrocarbons such as pentane, 2-methylhexane, cyclohexane, benzene, toluene, o—, m—, or p-xylene, and ethylbenzene, as well as aromatic or aliphatic ethers such as diphenyl ether, diethyl ether, p-dioxane, and tetrahydrofuran. Other solvents include aliphatic or aromatic ketones such as acetone, 4-methyl, 2-pentanone, and acetophenone. Still other suitable solvents include esters of carboxylic acids such as alkyl acetates, propionates, and octanoates. Especially preferred solvents are those esters which are formed as minor coproducts during normal practice of this invention, such as methyl acetate formed as a coproduct during reaction of methanol with carbon monoxide, and ethyl propionate, formed as a coproduct during reaction of carbon monoxide with ethanol. Other solvents include carboxylic acids such as acetic, propionic, octanoic, and isobutyric acids. Water could also be used as a solvent.

The temperature range which can usefully be employed in these syntheses is a variable dependent upon other experimental factors, including the pressure, the concentration and the choice of the particular species of soluble Group VIII metal catalyst, which in turn is in one embodiment a function of the composition of reactor and feed lines, among other things. The range of operability is from 240°C to 300°C, superatmospheric pressures of carbon monoxide being employed. It has been found that use of lower temperatures than used in prior art in conjunction with peat or coal-derived activated carbon sources of particular pore size produce an improvement in yield and selectivity and result in a greatly extended lifetime for the catalyst system over any noted in the prior art. Examples 12 and 16 will show catalyst lifetime of 96 to 218 hours, whereas the longest noted in previous systems is by the procedure of FR—A—2 030 118 and is less than 24 hours (Example 14).

Superatmospheric pressures of 3.5 MPa to 27.5 MPa lead to substantial yields of carboxylic acids by the process of this invention. The most preferred operating range is from 7 to 17.5 MPa.

In accordance with one embodiment of this invention, a spent carbonylation catalyst may be reactivated, e.g. after from 10 to 300 hours of use or more by a process comprising:

Contacting the deactivated carbon bed with steam at a temperature above 500°C, preferably 500 to 1500°C, and at a pressure of 0.1 to 0.5 MPa, optionally in the presence of activating agents, for a period of from 1 to 25, preferably 1 to 15 hours.

The activating agents can be selected from a large group of organic and inorganic compounds. It is only necessary that they aid in regenerating the porous structure of the deactivated carbon. Generally the activating agents used are alkali metals and alkaline earth metals and inorganic acids.

Suitable compounds containing alkali metals and alkaline earth metals include alkaline earth metal carbonates, alkaline earth metal oxides, alkaline earth metal hydroxides and alkaline earth metal halides. The alkali metal oxides, hydroxides, carbonates, and halides are also suitable. Effective compounds include $K_2O$, NaOH, KOH, $Ca(OH)_2$ $MgCl_2$, and BaO. Preferred is KOH.

Suitable inorganic acids used as activating agents in the process of the instant invention include phosphoric, sulfuric and boric acids.

Also suitable as activating agents are metal salts, typified by $ZnCl_2$ and $Na_2SO_4$; and inorganic sulfur compounds, typified by $K_2S$ and KNCS.

Various other suitable activating agents known in the art and expected to function in this invention are described in the book by A. Yehaskel, cited above.

The temperature range which can usefully be employed in the reactivation process is a variable dependent upon other experimental factors, including the pressure, the flow rate, the time of contact between the deactivated catalyst and the steam or water, the type of catalyst which was used in the carbon bed and the geometry of the catalyst bed. The range of operability is from 500 to 1500°C. A narrower range of 650 to 800°C represents the preferred temperature range.

Pressures of 0.01 to 0.5 MPa, and even greater lead to substantial reactivation of the depleted catalyst. A preferred operating range is from 0.1 to 0.5 MPa, although pressures above 0.5 MPa also provide reactivation.

The period of time necessary for the steam to contact the deactivated carbon catalyst in order to bring about reactivation may vary. For example, when steam is at a temperature of 705°C, it would appear a period of at least 3 hours of contact is desirable to reactivate the catalyst.

It is anticipated that a relationship exists wherein the higher the temperature and flow rate, the lower the contact time necessary.

In the process of the present invention, the amount of carbon monoxide present in the reaction mixture should be sufficient at least to satisfy the stoichiometry of the desired carbonylation reaction.

The major by-products of these acetic acid/ester syntheses are most commonly methyl propionate and propionic acid and derivatives, which are, of course, also useful compounds and major articles of commerce. The propionic acid and other products can easily be separated from one another by conventional means, e.g. fractional distillation in vacuo.

The products have been identified in this work by one or more of the following analytical procedures, viz, gas-liquid phase chromatograph (glc), infrared (ir), mass spectrometry, nuclear magnetic resonance (nmr) and elemental analyses, or a combination of these techniques. Analyses have, for the most part, been by parts in weight; all temperatures are in degrees centigrade and all pressures in MPa.

Having described the inventive process, the following examples are submitted to supply specific and illustrative embodiments.

Example 1

The example describes the use of ruthenium chloride as the soluble transition metal catalyst used in association with an activated carbon bed and an iodomethane promoter. As discussed earlier, the effectiveness of the components of the process of this invention allow the use of much lower concentrations of soluble Group VIII metal containing catalysts than used in processes in the prior art.

In this example, a 25 ml tubular reactor constructed of Hastealloy C equipped for downflow operation was packed with NORIT RB-1 Activated Carbon (which had been washed with aqueous $HNO_3$ at 100°C followed by deionized water, and finally vacuum drying). A solution consisting of 2432 g of methanol, 1197 g of iodomethane, and 0.200 g of $RuCl_3 \cdot H_2O$ was fed continuously at 6 ml/h, and CO was fed at 7000 ml/h to the reactor maintained at 275°C and 20.78 MPa. After 32 hours, analysis of liquid effluent indicated 97% of acetic acid, 2% of methyl acetate and 1% of $H_2O$. Virtually no dimethyl ether was detected.

Comparative Example 1A

This example was identical to Example 1, except operating conditions were changed so that the liquid feed rate was 12 ml/h and CO rate was 12000 ml/h. Typical acetic acid content of effluent under these conditions was 96%.

Comparative Example 1B

This example was identical to Examples 1 and 1A, except that operating conditions were changed so that the liquid feed rate was 20 ml/h and CO rate was approximately 20 ml/h. Liquid analysis indicated 11% of acetic acid. The feed was recovered. A black ruthenium-containing precipitate (16.0% Ru) was present and no detectable Ru was in solution (i.e. <1 ppm). Analysis of the initial solution indicated 26 ppm of Ru.

Examples 1, 1A and 1B illustrate the high productivity of the system when low concentrations of Ru are employed. They also demonstrate that activity diminishes as the ruthenium is removed by precipitation.

Effluent analyses averaged 6 ppm of Ru, which could be recovered if desired by concentrating the effluent and adding the concentrate to fresh MeOH/MeI feed. Analysis of the used carbon bed indicated approximately 0.6% of Ru.

Example 2

The procedure of Example 1 was repeated in essence, but a different ruthenium source, triruthenium-dodecacarbonyl $Ru_3(CO)_{12}$, was used, the concentration of ruthenium used being 18 ppm.

Operation under conditions indicated resulted in acetic acid content of the effluent as shown in Table 1:

TABLE 1

| Temp. (°C) | Pressure (MPa) | Liq. space velocity (ml/ml reactor vol/h) | Acetic acid (%) |
|---|---|---|---|
| 275 | 20.78 | 0.24 | 95.4 |
| 275 | 13.89 | 0.24 | 97.3 |
| 275 | 10.44 | 0.24 | 97.6 |
| 275 | 20.78 | 0.48 | 63.3 |
| 290 | 8.37 | 0.24 | 76.1 |

Duration of the run was 169 hours, final activity was high, as indicated by the last table entry. It may be noted that no precipitation of ruthenium occurred during three months storage of this feed at ambient conditions.

Example 3

Here, a cobalt-containing catalyst, $CO_2(CO)_8$, was used instead of $RuCl_3$, in essentially the same procedure as used in Example 1. Operation under conditions indicated resulted in acetic acid content of the effluent as shown in the following Table 2:

TABLE 2

| Temp. (°C) | Pressure (MPa) | Liq. space velocity (ml/h/ml reactor vol.) | Acetic acid (%) |
|---|---|---|---|
| 285 | 20.78 | 0.24 | 96.0 |
| 285 | 13.89 | 0.24 | 93.7 |
| 285 | 13.89 | 0.48 | 63.7 |
| 285 | 10.44 | 0.24 | 46.8 |

The run was 150 hours duration with activity manifested at termination as illustrated by the last table entry.

Comparative Example 4

This example demonstrates the unsuitability of a silicon-containing compound replacing the carbon bed in the process of this invention. The procedure of Example 3 was followed, except that a commercial tableted silica (Calisicat E-361) was used in place of the carbon bed. During 20 hours of operation at 20.78 MPa, 0.24 liquid hourly space velocity, and 285°C, the acetic acid content of the effluent was a maximum of 0.7%.

Comparative Example 5

In this example, the same procedure was followed as in Example 3, except that a commercial alumina (United CS.331-3) was used as packing. Maximum acetic acid content during a 20 hour run was 0.6%.

Example 6

In this example $Co_2(CO)_8$ was used, as in the procedure of Example 3, but the carbon was not washed. SORBONORIT B-3 was packed into the reactor in the form in which it was received, and was not washed. Maximum acetic acid content of the effluents was 97.2%, while operating at 19.75 MPa, 265°C and 0.24 liquid hourly space velocity.

Example 7

The procedure of Example 1 was followed, except that the liquid feed consisted of 1.48 g of $Fe(CO)_5$, 1218 g of methanol, and 593 g of iodomethane; the packing consisted of unwashed (as received) SORBONORIT B-3 carbon; operation during the 115 hour run and results are indicated below:

| Temp., °C | Pressure, MPa | ml liq./h/ml reactor | HOAc, % |
|---|---|---|---|
| 285 | 20.78 | 0.24 | 92 |
| 285 | 10.44 | 0.40 | 85 |

Example 8

This example illustrates a method by which soluble cobalt, where used as the catalyst compound, can be recovered for recycling to the feed solution.

The bulk of the effluents from examples 3 and 6 (440.8 g) was composited. The cobalt content of the composited sample before distillation was 20.2 ppm of Co. It was distilled at atmospheric pressure until 35.74 g of homogeneous dark liquid remained. Analysis indicated 287 ppm of Co, which if desired, could be recycled to the feed solution. Final distillation conditions were 117°C pot, 117°C head temp., 0.1 MPa. The liquid portion of the residue consisted of 99.3% of acetic acid, 0.2% of propionic acid, and 0.5% of water.

Comparative Example 9

This example is identical to Example 6 except that the reactor was constructed of tantalum rather than

7

Hastealloy C. Also no soluble metal was added to the liquid feed. Analysis of the feed sampled at a point immediately upstream of the reactor showed 5.6 ppm of nickel and 0.7 ppm of cobalt. Effluent analyses during operation at 285°C, 13.89 MPa and 0.40 liquid hourly space velocity indicated an acetic acid content which ranged from 24 to 34% during a 55 hour run.

Example 10

This example is identical to Example 9, except that a soluble nickel species, $NiI_2$, was introduced into the liquid feed by allowing the liquid feed (before entry into the reactor) to contact nickel metal alloys (stainless steel) during transit from the feed tank to the reactor. Analysis of the feed solution just upstream of the reactor showed 3 ppm of cobalt and 111 ppm of nickel. The acetic acid content of the effluent stream was generally between 85 and 90%.

Example 11

This example is identical to Example 9, except that 0.733 g of dicobalt octacarbonyl was added to 2750 ml of the liquid feed. Acetic acid content of the effluent stream was generally between 94 and 96%.

Example 12

Example 12 demonstrates the use of a peat-derived activated carbon, which is used at a temperature lower than that used in the prior art to produce an effluent which exceeded 90% of acetic acid, even after four days of continuous operation.

In this example, a peat-derived commercial grade activated carbon (NORIT RB-1) was washed with boiling 50% $HNO_3$. A 25 ml tubular reactor constructed of a nickel alloy (Hastealloy C) was filled with this carbon. A solution of 90 mole % methanol/10 mole % iodomethane was pumped continuously into the reactor at 10 ml/h while CO was simultaneously metered at 12000 ml/h. Reactor temperature and pressure were maintained at 285°C and 13.89 MPa.

Effluent analysis after 26 hours of operation, indicated 94.3% of acetic acid and 4.3% of methyl acetate. After 49 hours, the effluent consisted of 96.4% of acetic acid and 2.8% of methyl acetate. After four days of continuous operation, the acetic acid content of the effluent was still greater than 90%.

Example 13

This example demonstrates the results when an activated carbon is used which has approximately a 10% higher surface area than NORIT RB-1.

The procedure of Example 12 was followed, except that the activated carbon used was SORBONORIT B-3, (used as received from manufacturer). Effluent analysis after 18 hours indicated 95.1% of acetic acid and 2.9% of methyl acetate. After 42 hours, the effluent consisted of 95.3% of acetic acid and 3.1% of methyl acetate.

Example 14

In this example, the procedure of Example 13 was duplicated except that reaction conditions more similar to those of the prior art (i.e. FR—A—2 030 118) were used, in order to demonstrate by comparison the improvement in the process of this invention, wherein a longer catalyst lifetime is obtained by using lower temperatures and higher pressures, in conjunction with activated carbon sources containing a certain range of pore sizes.

In Example 14, the procedure of Example 13 was duplicated except that the reactor temperature was maintained at 315°C and the pressure was maintained at 8.37 MPa. The following Table 3 shows the effluent analysis after given periods of time.

TABLE 3

| Time (h) | Acetic acid content | Methyl acetate content |
|---|---|---|
| 16 | 84.4 | 6.0 |
| 23 | 3.9 | 2.8 |
| 40 | None | 1.7 |

Although acetic acid is produced in appreciable quantities initially, the catalyst rapidly becomes deactivated so that no acetic acid is formed after only 40 hours of operation.

Example 15

This example, in contrast to Example 14, demonstrates the improved results of this invention which are obtained using lower temperatures, i.e. lower than 300°C, but greater than 250°C.

Here, the procedure of Example 13 was duplicated except that the operating temperature was 250°C for 23 hours, and 265°C thereafter.

Analysis of effluent samples are shown below.

8

TABLE 4

| Total hours of operation | Temperature (°C) | Acetic acid content (%) | Methyl acetate content (%) |
|---|---|---|---|
| 22 | 250 | 13.9 | 7.9 |
| 50 | 265 | 45.8 | 19.8 |

This example, and the results of Example 13, demonstrate the advisability of conducting the reaction at above 265°C, but below 300°C to achieve high selectivity to acetic acid.

Example 16

This example demonstrates the results when an activated carbon is used which is derived from coal and which has wide pores.

The procedure of Example 12 was duplicated except that the activated carbon used was CARBORUNDUM GAC 616GA. Effluent analyses are indicated below after various periods of continuous operation.

| Hours of operation | % Acetic acid content | % Methyl acetate content |
|---|---|---|
| 46 | 94 | 3 |
| 72 | 98 | 1 |
| 144 | 98 | 1 |
| 164 | 94 | 5 |
| 188 | 90 | 8 |
| 218 | 70 | 15 |

It can be noted that after 218 hours of continuous operation, high conversions of methanol, and high selectivity to acetic acid and methyl acetate were being obtained.

Examples 17 to 20

Examples 17 to 20 show variations in results obtained by reacting carbon monoxide and methanol over activated carbons of various origins. The procedure of Example 13 was duplicated using various kinds of activated carbon as shown in the table below. Also shown are effluent compositions after various durations:

| Example | Carbon | Hours | % Acetic acid | % Methyl acetate |
|---|---|---|---|---|
| 17 | Witcarb Columbia JXC Petroleum Origin | 22 | 47 | 9 |
| | | 63 | 0 | 2 |
| 18 | North American G-212 Coconut Shell Origin | 24 | 38 | 24 |
| 19 | Carborundum GAC 616G Coal Origin | 23 | 20 | 5 |
| | | 44 | 14 | 4 |
| 20 | Arrow briquettes Hardwood Sawdust Origin | 18 | 78 | 15 |
| | Activated at 720°C with Steam and HCl | 24 | 21 | 5 |

These examples and the results of Examples 12 and 13 indicated peat-derived carbons are superior to non peat-derived.

Example 21

This example describes the initial use of the carbon catalyst beds of SORBONORIT B-3 and demonstrates the effect on spent catalysts of using the reactivation process of this invention.

9

a) The 25 ml tubular reactor constructed of nickel alloy (Hastealloy C) was packed with SORBONORIT B-3 activated carbon (a peat-derived carbon of approximately 1000 m²/g surface area). A solution of 90 mole % of methanol/10 mole % of iodomethane was pumped continuously to the reactor at 10 ml/h while CO was simultaneously metered at 12000 ml/h. Reactor temperature and pressure were maintained at 285°C and 13.89 MPa. After 42 hours the effluent comprised 95.3% of acetic acid and 3.1% of methyl acetate. After 88 hours, activity had declined, so that effluent comprised 95% of $H_2O$/methanol, 4.7% of methyl iodide/methyl acetate, and only 0.5% of acetic acid.

b) The above procedure was duplicated, except that the reactor temperature was maintained at 315°C and the pressure was maintained at 8.37 MPa. After 40 hours of continuous operation, activity had declined to yield only 1.7% of methyl acetate and no acetic acid in the effluent.

c) The above procedure was duplicated, except that the operating temperature was 250°C for 23 hours, and 265°C for an additional 96 hours, with pressure maintained at 13.89 MPa. At the end of the operating period, effluent acetic acid content was 10.2%, whereas maximum acetic acid content (at 50 total hours) of operation) was 46%.

d) The middle portions of the used catalyst beds from the 3 procedures described were combined and mixed well. A 45 ml aliquot was charged to a quartz tube and heated in a stream of flowing nitrogen to 705°C. Thereafter $H_2O$ was charged additionally in a continuous manner at 0.191 ml/min while the temperature in the regeneration zone was maintained at 710±10°C for 7 hours.

Example 22

The procedure of Example 21(b) was repeated using the resulting reactivated carbon (25 ml) of Example 21(d), with reactor temperature maintained at 285°C and pressure maintained at 13.89 MPa. High selectivity and activity to acetic acid were exhibited.

At 26 hours, the effluent contained 95.2% of acetic acid. After 120 hours, the effluent contained 11.2% of acetic acid, and after 141 hours, it contained only 0.8% of acetic acid.

Example 23

This example illustrates the use of twice reactivated SORBONORIT B-3 carbon. Here, the bulk of the carbon bed used in Example 22 was subjected to the identical reactivation procedure of Example 21(d). Then the procedure of Example 22 was repeated, using 24 ml of the twice-reactivated carbon material. The reactor was operated for 24 hours, and at 18 hours of operation the effluent showed 92.0% of acetic acid.

Example 24

This example demonstrates the use of the process for reactivation when the carbon has been used in the presence of a cobalt catalyst.

a) The procedure of Example 21 was followed except that 111 ppm of Co (as $Co_2(CO)_8$) was present in the liquid feed, and acid-washed NORIT RB-1 activated carbon was used at 285°C and 13.89 MPa for 112.5 hours. The effluent contained more than 95% of acetic acid near the beginning of the run (e.g. 24 hours after start-up) and only 0.7% of acetic acid near the end of the reaction period.

b) The procedure of this Example was repeated, except that operation was for 140 hours at 285°C and 13.89 MPa and then the temperature was raised to 310°C, the liquid flow was decreased to 6 ml/h, and the gas flow was decreased to 7000 ml/h; this operation continued for 6 more hours. At the end of the 146 hour run, the effluent contained 5.3% of acetic acid.

c) The carbon described above in this example was left in the reactor. The reactor temperature was maintained at 360°C, and the pressure at 1.48 MPa while $H_2O$ was introduced at 4 ml/h for 19 hours. Use of this carbon at 13.89 MPa and 285°C as described above produced effluent which showed no acetic acid.

d) The middle portions of the two used catalyst beds of this example were combined, mixed well, and placed in a quartz tube. Regeneration was conducted at 705 to 710°C with $H_2O$ fed at 0.191 ml/min for 2½ hours and at 0.0764 ml/min for 1 hour more in a stream of $N_2$. 32 ml of carbon were initially charged, and 32 ml were recovered.

e) The procedure of Example 22 was conducted using this regenerated carbon. Effluent collected during the first 16 hours contained 14.3% of acetic acid and 6.8% of methyl acetate. Effluent collected thereafter contained no acetic acid.

This example illustrates that the milder regeneration procedure is not so effective as that used in Example 21.

**Claims**

1. A process for the continuous production of carboxylic acids and esters by the reaction of carbon monoxide with an alkanol at an elevated temperature and pressure in the presence of a halide promoter, activated carbon, and in the presence of at least 1 ppm of a transition metal selected from cobalt, ruthenium, iron, nickel, rhodium, palladium, osmium, iridium and platinum, characterized in that the activated carbon is derived from coal or peat and has a density from 0.03 to 2.5 cm³/g, a surface area from 200 to 2000 m²/g, and a pore size from 0.6 to 200 nm, and in that the temperature is from 240 to 300°C and the pressure is from 3.5 to 27.5 MPa.

2. A process according to Claim 1 characterized in that said reaction takes place at a pressure from 5 to 27.5 MPa in the presence of a halide, carboxylate, nitrate or carbonyl of the transition metal.

3. A process according to Claim 1 characterized in that said reaction takes place at a pressure of from 3.5 to 21 MPa, the transition metal resulting from the reaction being conducted in a nickel or cobalt alloy reactor.

4. A process according to any of the preceding Claims characterized in that the amount of transition metal is from 25 to 150 ppm.

5. A process according to any of the preceding Claims characterized in that the halide promoter is an alkyl halide, hydrogen halide, acyl halide or dihalomethane.

6. A process according to any of the preceding Claims characterized in that the reaction is carried out in the presence of a solvent.

7. A process according to any of the preceding Claims characterized in that the catalyst is reactivated by contact with steam at a temperature of from 500 to 1500°C and a pressure of from 0.1 to 0.5 MPa for a period of from 1 to 25 hours.

8. A process according to Claim 7 characterized in that the catalyst reactivation is conducted in the presence of an activating agent selected from oxides, hydroxides and halides of alkali metals or alkaline earth metals, inorganic sulphur compounds and mineral acids.

## Patentansprüche

1. Verfahren zur kontinuierlichen Herstellung von Carbonsäuren und Estern durch Umsetzung von Kohlenmonoxid mit einem Alkanol bei erhöhter Temperatur und Druck in Gegenwart eines Halogenid-Promotors, Aktivkohle, und in Gegenwart von mindestens 1 ppm eines der folgenden Übergangsmetalle Kobalt, Ruthenium, Eisen, Nickel, Rhodium, Palladium, Osmium, Iridium und Platin, dadurch gekennzeichnet, daß die Aktivkohle von Kohle oder Torf stammt und eine Dichte von 0,03 bis 2,5 cm$^3$/g, eine Oberfläche von 200 bis 2000 m$^2$/g und eine Porengröße von 0,6 bis 200 nm aufweist und daß die Temperatur 240 bis 300°C und der Druck 3,5 bis 27,5 MPa betragen.

2. Verfahren nach Anspruch 1, dadurch gekennzeichnet, daß die Reaktion bei einem Druck von 5 bis 27,5 MPa in Gegenwart eines Halogenids, Carboxylats, Nitrats oder Carbonyls des Übergangsmetalls durchgeführt wird.

3. Verfahren nach Anspruch 1, dadurch gekennzeichnet, daß die Reaktion bei einem Druck von 3,5 bis 21 MPa durchgeführt wird und das Übergangsmetall dadurch bereitgestellt wird, daß die Umsetzung in einem Reaktor durchgeführt wird, der aus einer Nickel- oder Kobaltlegierung besteht.

4. Verfahren nach einem der vorhergehenden Ansprüche, dadurch gekennzeichnet, daß die Menge des Übergangsmetalls 25 bis 150 ppm beträgt.

5. Verfahren nach einem der vorhergehenden Ansprüche, dadurch gekennzeichnet, daß der Halogenid-Promotor ein Alkylhalogenid, Wasserstoffhalogenid, Acylhalogenid oder ein Dihalogenmethan ist.

6. Verfahren nach einem der vorhergehenden Ansprüche, dadurch gekennzeichnet, daß die Umsetzung in Gegenwart eines Lösungsmittels durchgeführt wird.

7. Verfahren nach einem der vorhergehenden Ansprüche, dadurch gekennzeichnet, daß der Katalysator durch Kontakt mit Wasserdampf bei einer Temperatur von 500 bis 1500°C und einem Druck von 0,1 bis 0,5 MPa für einen Zeitraum von 1 bis 25 Stunden reaktiviert wird.

8. Verfahren nach Anspruch 7, dadurch gekennzeichnet, daß die Reaktivierung des Katalysators in Gegenwart eines Aktivierungsmittels durchgeführt wird, nämlich in Gegenwart von Oxiden, Hydroxiden und Halogeniden von Alkalimetallen oder Erdalkalimetallen, anorganischen Schwefelverbindungen oder Mineralsäuren.

## Revendications

1. Procédé pour la préparation continue d'acides et d'esters carboxyliques par réaction de l'oxyde de carbone avec un alcanol à une température et sous une pression élevées en présence d'un halogénure comme promoteur, de carbone activé, et en présence d'au moins 1 ppm d'un métal de transition choisi parmi le cobalt, le ruthénium, le fer, le nickel, le rhodium, le palladium, l'osmium, l'iridium et le platine, caractérisé en ce que le carbone activé est obtenu à partir de charbon ou de tourbe et a une densité de 0,03 à 2,5 cm$^3$/g, une surface spécifique de 200 à 2000 m$^2$/g et une taille de pores de 0,6 à 200 nm, et en ce que la température est de 240 à 300°C et la pression est de 3,5 à 27,5 MPa.

2. Procédé suivant la revendication 1, caractérisé en ce que cette réaction s'effectue sous une pression de 5 à 27,5 MPa en présence d'un halogénure, carboxylate, nitrate ou composé carbonylé du métal de transition.

3. Procédé suivant la revendication 1, caractérisé en ce que cette réaction s'effectue sous une pression de 3,5 à 21 MPa, le métal de transition provenant de ce que la réaction est effectuée dans un réacteur d'alliage de nickel ou de cobalt.

4. Procédé suivant l'une quelconque des revendications précédentes, caractérisé en ce que la quantité de métal de transition est de 25 à 150 ppm.

5. Procédé suivant l'une quelconque des revendications précédentes, caractérisé en ce que l'halogénure promoteur est un halogénure d'alkyle, un halogénure d'hydrogène, un halogénure d'acyle ou un dihalométhane.

6. Procédé suivant l'une quelconque des revendications précédentes, caractérisé en ce que la réaction est effectuée en présence d'un solvant.

7. Procédé suivant l'une quelconque des revendications précédentes, caractérisé en ce que le catalyseur est réactivé par contact avec de la vapeur à une température de 500 à 1500°C et sous une pression de 0,1 à 0,5 MPa pendant une durée de 1 à 25 h.

8. Procédé suivant la revendication 7, caractérisé en ce que la réactivation du catalyseur est effectuée en présence d'un agent d'activation choisi parmi les oxydes, hydroxydes et halogénures de métaux alcalins ou de métaux alcalinoterreux, les composés minéraux du soufre et les acides minéraux.